# EUROPÄISCHE PATENTSCHRIFT

(11) **EP 2 708 876 B1**
(45) Veröffentlichungstag und Bekanntmachung des Hinweises auf die Patenterteilung: **06.02.2019**
(21) Anmeldenummer: 13178519.8
(22) Anmeldetag: 30.07.2013
(51) Int. Cl.: B01L 3/00, G01N 33/543, C12Q 1/6825, H01L 21/02, H01L 29/66

(54) **Verfahren zum Herstellen einer Sensorvorrichtung zum Detektieren von chemischen oder biologischen Spezies und Verfahren zum Herstellen einer mikrofluidischen Vorrichtung mit einer derartigen Sensorvorrichtung.**
Method for producing a sensor device for detecting chemical or biological species and method for producing a microfluidic device with such a sensor device.
Procédé de fabrication d'un dispositif de capteur électronique pour détecter des espèces chimiques ou biologiques et procédé de fabrication d'un dispositif microfluidique comportant un tel dispositif de capteur.

(30) Priorität: 17.09.2012 DE 102012216497
(43) Veröffentlichungstag der Anmeldung: 19.03.2014
(73) Patentinhaber: ROBERT BOSCH GMBH, 70442 Stuttgart (DE)
(72) Erfinder: Bischopink, Georg, 72124 Pliezhausen (DE); Brettschneider, Thomas, 71229 Leonberg (DE); Laermer, Franz, 71263 Weil Der Stadt (DE); Dorrer, Christian, 70193 Stuttgart (DE)

(56) Entgegenhaltungen:
- EP-A2- 1 591 780
- WO-A1-2010/089226
- DE-A1- 10 111 458
- DE-A1- 10 122 133
- US-A1- 2004 000 713
- US-A1- 2007 122 314
- BRAUN T ET AL: "Large area embedding for heterogeneous system integration", ELECTRONIC COMPONENTS AND TECHNOLOGY CONFERENCE (ECTC), 2010 PROCEEDINGS 60TH, IEEE, PISCATAWAY, NJ, USA, 1. Juni 2010 (2010-06-01), Seiten 550-556, XP031694139, ISBN: 978-1-4244-6410-4

## Beschreibung

Die Erfindung bezieht sich auf eine elektronische Sensorvorrichtung zum Detektieren von chemischen oder biologischen Spezies, eine mikrofluidische Vorrichtung mit einer derartigen Sensorvorrichtung, ein Verfahren zum Herstellen einer derartigen Sensorvorrichtung sowie ein Verfahren zum Herstellen einer derartigen mikrofluidischen Vorrichtung.

### Stand der Technik

Es sind Chemo- und Biosensoren bekannt, bei denen auf einem Siliziumsubstrat ein Feldeffekttransistor realisiert ist, dessen Gate eine Detektionselektrode des Sensors bildet, wie beispielsweise in KR 10 2010 00100083 A und EP 2 378 559 A1 offenbart.

Auch sind Chemo- bzw. Biosensoren bekannt, bei denen auf einem Siliziumsubstrat eine Elektrode aus Gold abgeschieden wird, wie beispielsweise in US 7 776 794 B2 offenbart

Ebenfalls sind als polymere Mehrschichtanordnungen aufgebaute mikrofluidische Systeme mit darin integrierten Chemo- bzw. Biosensoren bekannt, wie beispielsweise in US 2007/0122314 A1 offenbart.

US 2004/0200734 A1 offenbart einen Sensor zum Detektieren von Biomolekülen, beispielsweise Proteinen, Nukleinsäuren oder Antikörpern. Der Sensor umfasst Nanoröhren, die mit zwei Elektroden verbunden und in ein mikrofluidisches System integriert sind, und in den Nanoröhren immobilisierte Biomoleküle. Dieser Sensor minimalisiert das Reagenzvolumen und ermöglicht ein rasches Screening mit hohem Durchsatz von potenziel wirksamen Medikamentverbindungen.

WO 2009/014390 A2 offenbart einen mikrofluidischen Sensoraufbau zur quantitativen Analyse von Proben, wie etwa Myoglobin und Antibiotika. Der Aufbau umfasst eine untere Platte, eine mittlere Platte und eine obere Platte. Auf der unteren Platte ist eine Referenzelektrode, eine Arbeitselektrode und ein Elektrodenanschluss ausgebildet. Die mittlere Platte wird auf die untere Platte aufgelegt und umfasst einen Probeneinlasskanal, einen mikrofluidischen Kanaldurchlass, der sich von dem Probeneinlasskanal aus erstreckt und der als eine Führung für den Probenfluss über die gesamte mittlere Platte dient und der sich an einer Position in der Nähe des Probeneinlasskanals in zwei Teilkanäle verzweigt. In einem Teilkanal ist ein Enzym-Konjugat-Reservoir und in dem anderen Zweig ein Substrat-Reservoir vorgesehen. Die zwei Teilkanäle fließen stromaufwärts eines Nachweiskanals zusammen, in dessen Bereich die Referenzelektrode und die Arbeitselektrode auf der ersten Platte bloßliegen. Die mittlere Platte weist ferner einen Mischkanal auf, der an einer Position des Zusammenflusses stromabwärts des Substrat-Reservoirs angeordnet ist und der einen Luftauslasskanal aufweist, sodass eine Probe, die durch das Substrat-Reservoir hindurchströmt, den Nachweiskanal später erreicht als eine Probe, die durch das Enzym-Konjugat-Reservoir strömt. Schließlich weist die mittlere Platte noch einen Absorptionskanal auf, in dem das aus dem Detektionskanal ausströmende Probenfluid absorbiert wird, und einen Lufteinlasskanal, der in ein Ende des Absorptionskanals mündet.

Weitere Beispiele für Chemo- und Biosensoren sind in der DE 101 22 133 A1, der US 2004/0000713 A1, der WO 2010/089226 A1 und der DE 101 11 458 A1 beschrieben.

### Offenbarung der Erfindung

Die Erfindung schafft ein Verfahren zum Herstellen einer Vielzahl von elektronischen Sensorvorrichtungen mit den Merkmalen des Anspruchs 1 sowie ein Verfahren zum Herstellen einer mikrofluidischen Vorrichtung zum Detektieren von chemischen oder biologischen Spezies mit den Merkmalen des Anspruchs 2.

### Kurze Beschreibung der Figuren

Die Erfindung wird im Folgenden beispielhaft anhand von auf der Grundlage der beigefügten Figuren beschriebenen Ausführungsformen des erfindungsgemäßen elektronischen Sensors, der mikrofluidischen Vorrichtung und Ausführungsformen von Verfahren zu deren Herstellung in weiteren Einzelheiten beschrieben. Es zeigen:
Fig. 1 eine schematische perspektivische Ansicht einer elektronischen Sensorvorrichtung, welche mittels des erfindungsgemäßen Verfahrens hergestellt ist;
Fig. 2 eine schematische Querschnittansicht der elektronischen Sensorvorrichtung aus der Fig. 1 und eine Detailvergrößerung dieser Querschnittansicht; und
Fig. 3 eine schematische Querschnittsansicht einer Ausführungsform einer mikrofluidischen Vorrichtung, in der die Sensorvorrichtung aus der Fig. 1 integriert ist.

### Ausführungsformen der Erfindung

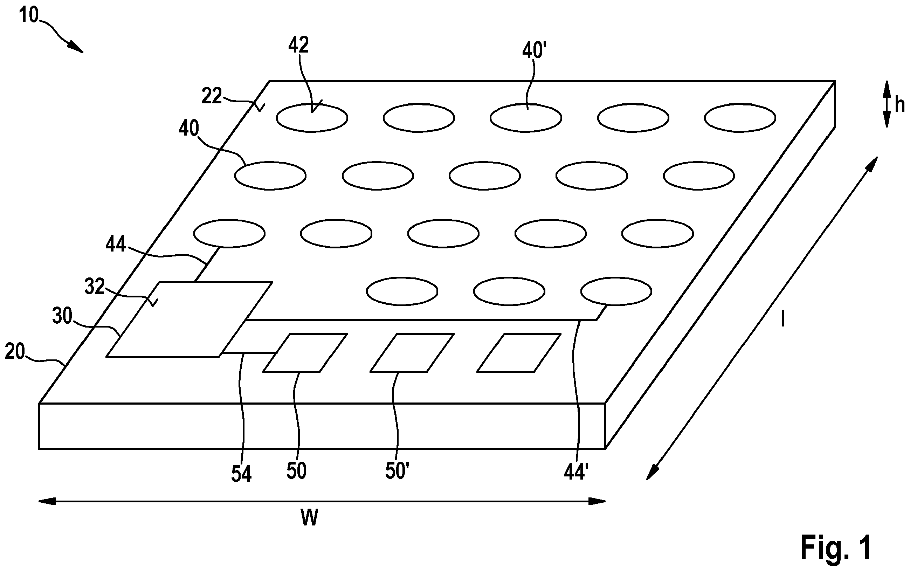

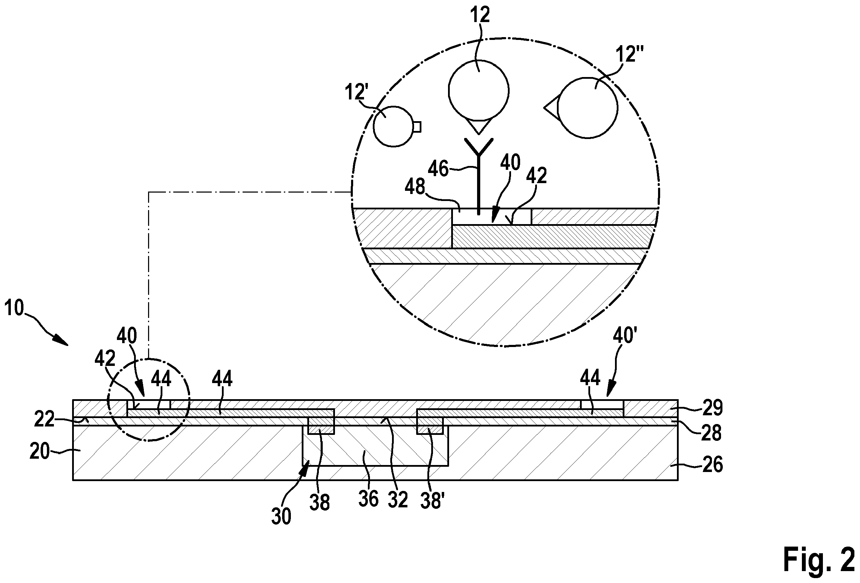

In der in Fig. 1 gezeigten elektronischen Sensorvorrichtung 10 umfasst diese ein aus einem schmelzumformbaren Material (Moldmasse) hergestelltes Substrat 20 mit einer Substratoberfläche 22, einen aus einem Siliziumsubstrat 36 vorab hergestellten Halbleiter-Chip 30 (siehe auch Fig. 2, untere Abbildung), der in einem ersten Bereich der Substratoberfläche 22 aufgebracht, eingebettet oder zumindest teilweise eingebettet ist, eine Vielzahl von Sensoreinrichtungen 40, 40', die in einer Vielzahl von entsprechenden jeweiligen zweiten Bereichen auf der Substratoberfläche 22 aufgebracht, eingebettet oder zumindest teilweise eingebettet sind, eine entsprechende Vielzahl von elektrisch leitfähigen Verbindungen 44, 44', die auf der Substratoberfläche 22 aufgebracht sind und eine elektrisch leitfähige Verbindung zwischen einer jeweiligen Sensoreinrichtung 40, 44 und dem Halbleiter-Chip 30 ausbilden. Die elektronische Sensorvorrichtung 10 umfasst ferner eine Vielzahl von Kontaktierungsschichten 50, 50', die in jeweiligen dritten Bereichen auf der Substratoberfläche 22 aufgebracht oder zumindest teilweise eingebettet sind und eine entsprechende Vielzahl von leitfähigen Verbindungen 54, die auf der Substratoberfläche 22 aufgebracht oder zumindest teilweise eingebettet sind und die eine elektrisch leitfähige Verbindung zwischen einer jeweiligen Kontaktierungsschicht 50, 50' und dem Halbleiter-Chip 30 ausbilden.

Der Halbleiter-Chip 30 wurde mit aus dem Bereich der Halbleitertechnologie bekannten Herstellungsprozessen vorgefertigt. Der Halbleiter-Chip 30 umfasst mindestens eine Einrichtung zum Verstärken von Spannungen, Strömen, elektrischen Ladungen und/oder zum Auslesen von Kapazitätsänderungen, die in einer der Sensoreinrichtungen 40, 40' im Einsatz infolge einer Detektion einer chemischen oder biologischen Spezies 12, 12', 12" (siehe Fig. 2, obere Abbildung) in der Sensoreinrichtung 40, 40' erzeugt werden können. Der Halbleiter-Chip 30 umfasst ferner einen Mikrocontroller zur Steuerung der Verstärkungseinrichtung/en. Der Halbleiter-Chip 30 ist als anwendungsspezifische Schaltung (ASIC, englisch: Application Specific Integrated Circuit) ausgebildet.

Die übrigen elektrischen Elemente 40, 40', 44, 50, 50', 54 werden mit Dünnschichttechnologien oder mittels Druckverfahren, beispielsweise Siebdruck-, Nadeldruck- oder Tintenstrahl-Verfahren, auf die Substratoberfläche 22 des schmelzumformbaren Materials (Moldmasse) erzeugt bzw. aufgebracht. In Fig. 1 sind zur Vereinfachung der Darstellung nicht alle von dem Halbleiter-Chip 30 ausgehenden und zu den jeweiligen Sensoreinrichtungen 40, 40' führenden elektrisch leitfähigen Verbindungen 44 und auch nicht alle von dem Halbleiter-Chip 30 ausgehenden und zu den jeweiligen Kontaktierungsschichten 50, 50' führenden elektrisch leitfähigen Verbindungen 54 gezeigt.

Die Sensoreinrichtungen (z.B. Elektroden) 40, 40' weisen jeweils eine aktive Sensoroberfläche 42 auf, auf der eine Beschichtung aufgebracht ist, die es erlaubt, bei Kontakt mit einer Analytlösung selektiv bestimmte chemische oder biologische Spezies 12, 12', 12", wie etwa Ionen, Moleküle, Proteine, RNA-Moleküle, DNA-Sequenzen, Antigene, Viren, Bakterien oder Zellen, zu detektieren. Dazu wurden auf den aktiven Sensoroberflächen 42 in einer Ausführungsform bestimmte Fängermoleküle 46, wie etwa Antikörper oder DNA-Fragmente, aufgebracht und ggf. beispielsweise mittels geeigneter Verbindungsmoleküle 48 (vom Fachmann auch als Linker bzw. Linker-Moleküle bezeichnet) auf der aktiven Sensoroberfläche 42 immobilisiert. In einer dazu alternativen Ausführungsform kann auf der aktiven Sensoroberfläche 42 auch eine ionenselektive Schicht aufgebracht sein, siehe Fig. 2, obere Abbildung (Ausschnittvergrößerung).

Fig. 2 zeigt eine vereinzelte elektronische Sensorvorrichtung 10 im Querschnitt. Zur Herstellung einer Vielzahl derartiger elektronischer Sensorvorrichtungen 10 in einem parallel verarbeitenden Herstellungsprozess wurde eine Vielzahl von vorgefertigten Halbleiter-Chips 30 auf einer Klebefolie (nicht gezeigt) positioniert, wobei die Chipoberfläche 32 der Klebefolie zugewandt ist, und mit einem schmelzformbaren Material (Moldmasse) umspritzt und auf diese Weise in das schmelzformbare Material 26 eingebettet. Danach wurde die Klebefolie abgenommen, sodass man ein Substrat aus schmelzumformbarem Material 26 mit einer Vielzahl darin eingebetteter Halbleiter-Chips 30 erhält. Auf diesem Moldmassen-Substrat wurde ein erster Fotolack als Fotolack-Passivierungsschicht 28 aufgebracht und anschließend über vorbestimmten Kontaktflächen 38, 38' auf den Chipoberflächen 32 der Halbleiter-Chips 30 geöffnet. Dann werden auf der Fotolack-Passivierungsschicht 28 die elektrisch leitfähigen Verbindungen 44, 44' und die dadurch mit den Halbleiter-Chips 30 elektrisch leitfähig verbundenen Sensoreinrichtungen 40, 40', die als Elektroden ausgebildet sein können, erstellt.

Zur Herstellung der elektrisch leitfähigen Verbindungen 44, 44' und/oder der Sensoreinrichtungen 40, 40' können galvanische Methoden verwendet und damit elektrisch leitfähige Materialien, insbesondere Metalle, beispielsweise Kupfer, Aluminium, Silber oder Gold, aufgebracht werden.

Auf der Fotolack-Passivierungsschicht 28 werden die elektrisch leitfähigen Verbindungen 54 und die dadurch mit den Halbleiter-Chips 30 elektrisch leitfähig verbundenen Kontaktierungsschichten 50, 50' aufgebracht. Dafür können ebenfalls galvanische Methoden verwendet werden.

Danach wird ein zweiter Fotolack als Fotolack-Stoppschicht 29 aufgebracht. Diese Stoppschicht 29 wird nur über den Sensoreinrichtungen 40, 40', d.h. in den zweiten Bereichen, und über den Kontaktierungsschichten 50, 50' geöffnet.

Die Sensoreinrichtungen 40, 40' und/oder die Kontaktierungsschichten 50, 50' werden in nachfolgenden Prozessen mit jeweiligen weiteren Schichten belegt. Beispielsweise können die Kontaktierungsflächen 50, 50' mit Gold belegt werden. Insbesondere werden die Sensoreinrichtungen 40, 40' mit der Beschichtung belegt, die es erlaubt, bei Kontakt mit einer Analytlösung selektiv bestimmte chemische oder biologische Spezies 12, 12', 12" zu detektieren. Dabei können verschiedene Sensoreinrichtungen 40, 40' oder Gruppen von Sensoreinrichtungen mit unterschiedlichen, zur Detektion unterschiedlicher vorbestimmter chemischer oder biologischer Spezies geeigneter Beschichtungen funktionalisiert werden, sodass mit den unterschiedlich funktionalisierten Sensoreinrichtungen 40, 40' unterschiedliche chemische oder biologische Spezies detektiert werden können.

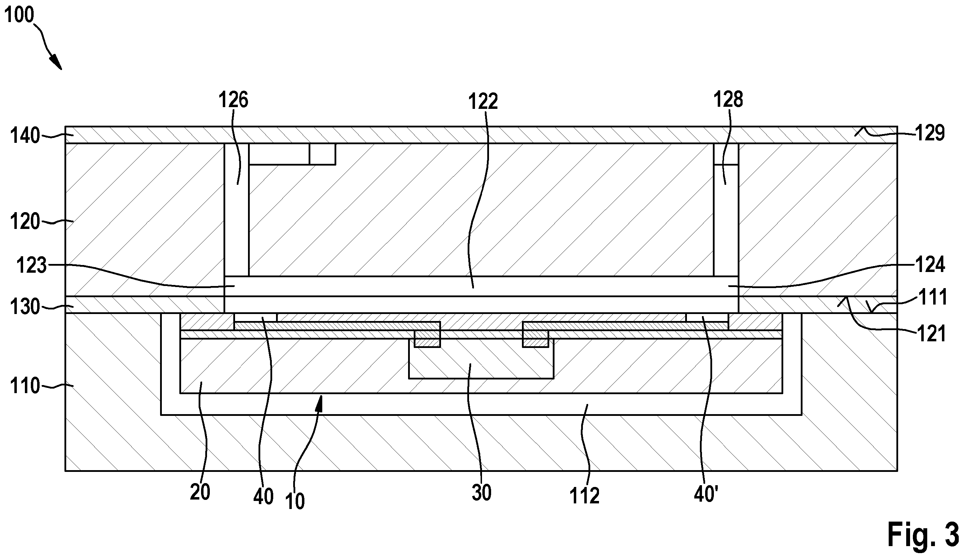

Fig. 3 zeigt eine Ausführungsform der Integration einer elektronischen Sensorvorrichtung 10 aus der Fig. 1 in eine mikrofluidische Vorrichtung 100. Letztere umfasst einem mehrschichtigen Aufbau mit zwei strukturierten Polymersubstraten 110, 120.

Das erste (in Fig. 3 untere) Polymersubstrat 110 weist eine strukturierte Oberfläche 111 auf, in der eine Ausnehmung 112 ausgebildet ist. In dieser Ausnehmung 112 ist eine elektronische Sensorvorrichtung 10 so aufgenommen, dass die obere Oberfläche der elektronischen Sensorvorrichtung 10 bündig mit der Oberfläche 111 des ersten Polymersubstrats 110 in den Bereichen außerhalb der Ausnehmung 112 angeordnet ist.

Das zweite (in Fig. 3 obere) Polymersubstrat 120 weist eine strukturierte bzw. strukturierbare Oberfläche 121 auf, die dem ersten Polymersubstrat 110 zugewandt ist. In der Oberfläche 121 ist eine Flusszelle 122 ausgebildet, beispielsweise ein Kanal, der sich in dem Bereich der Sensoreinrichtungen 40, 40' des in der Ausnehmung 112 im ersten Polymersubstrat 110 eingebetteten elektronischen Sensoreinrichtung 10 erstreckt. Der Flusszelle 122 bzw. dem Kanal zugeordnet ist eine Zuflussleitung 126 zum Zuführen eines Analyten und/oder einer Waschlösung. Dabei mündet die Zuflussleitung 126 in einem ersten Endabschnitt 123 der Flusszelle 122. Der Flusszelle 122 bzw. dem Kanal ist auch eine Abflussleitung 128 zum Ableiten des Analyten bzw. der Waschlösung zugeordnet. Die Abflussleitung 128 mündet in einem zweiten Endabschnitt 124 der Flusszelle 122 bzw. des Kanals.

Die elektronische Sensorvorrichtung 10 ist mit dem zweiten Polymersubstrat in Bereichen außerhalb der Flusszelle 122 flächig verbunden. Das zweite (in Fig. 3 obere) Polymersubstrat 120 ist mit dem ersten (in Fig. 3 unteren) Polymersubstrat 110 in Bereichen außerhalb der Ausnehmung 112 in der Oberfläche 111 des ersten Polymersubstrats 110 flächig verbunden. Zur flächigen Verbindung ist eine strukturierte Klebefolie 130, beispielsweise eine Schmelzklebefolie, vorgesehen. Die Klebefolie 130 bewirkt die flächige Verbindung der elektronischen Sensorvorrichtung 10 mit dem zweiten Polymersubstrat 120 im Bereich außerhalb der Flusszelle 122 und die flächige Verbindung zwischen dem zweiten Polymersubstrat 120 und dem ersten Polymersubstrat 110 im Bereich außerhalb der Ausnehmung 112.

Alternativ zu der Verbindung mittels der Klebefolie 130 können diese Verbindungen bzw. die flächige Verbindung auch durch Schweißen, etwa durch Thermokompressionsschweißen oder Laserschweißen bewirkt werden.

Auf einer der strukturierten Oberfläche 121 abgewandten Oberfläche 129 des zweiten Polymersubstrats 120 ist noch eine Deckschicht 140 aufgebracht zum Schützen der Eingänge der Zuflussleitung 126 und der Abflussleitung 128. Die Deckschicht 140 kann als eine weitere Klebefolie oder als eine Polymerplatte ausgebildet sein.

Zusätzlich zu der elektronischen Sensorvorrichtung 10 umfasst die mikrofluidische Vorrichtung 100 noch weitere mikrofluidische Elemente (nicht gezeigt), wie etwa Ventile, Pumpen, Kammern oder Mischeinrichtungen, die zum Beschicken, Betreiben und Reinigen der aktiven Oberflächen der Sensorvorrichtung 10 benötigt werden.

Zur elektrischen Versorgung der in die mikrofluidische Vorrichtung 100 integrierten elektronischen Sensorvorrichtung 10 wird die Sensorvorrichtung 10, insbesondere über deren Kontaktierungsschichten 50, 50', beispielsweise über Federkontaktstifte (nicht gezeigt) oder gleichfunktionelle andere Kontakteinrichtungen (nicht gezeigt) mit in der mikrofluidischen Vorrichtung integrierten Leiterbahnen oder Metall- bzw. Drahtbonds (nicht gezeigt) kontaktiert.

Die elektronische Sensorvorrichtung 10 bzw. die damit bestückte mikrofluidische Vorrichtung 100 kann in analytischen Systemen, insbesondere für mikrofluidische Lab-on-Chip-Systeme, für die Umweltanalytik oder die medizinische Diagnostik verwendet werden.

Obwohl die vorher beschriebenen Ausführungsformen ein Siliziumsubstrat als Halbleiter-Chip verwenden, ist die Erfindung nicht darauf beschränkt, sondern für beliebige Halbleiter-Chips anwendbar.

### Bezugszeichenliste:

- 10: Sensorvorrichtung
- 12, 12', 12": chemische oder biologische Spezies
- 20: Substrat
- 22: Substratoberfläche
- 26: schmelzformbares Material
- 28: Photolack-Passivierungsschicht
- 29: Photolack-Stoppschicht
- w: Breite des Substrats
- l: Länge des Substrats
- h: Dicke des Substrats
- 30: Halbleiter-Chip
- 32: Halbleiter-Chipoberfläche
- 36: Halbleiter-Substrat
- 38, 38': Kontaktfläche
- 40, 40': Sensoreinrichtung
- 42: aktive Sensoroberfläche
- 44, 44': elektrisch leitfähige Verbindung
- 46: Fängermolekül
- 48: Linkermolekül
- 50, 50': Kontaktierungsschicht
- 54: elektrisch leitfähige Verbindung
- 100: mikrofluidische Vorrichtung
- 110: erstes Polymersubstrat
- 111: strukturierte bzw. strukturierbare Oberfläche
- 112: Ausnehmung
- 120: zweites Polymersubstrat
- 121: strukturierte bzw. strukturierbare Oberfläche
- 122: Flusszelle
- 123: erster Endabschnitt
- 124: zweiter Endabschnitt
- 126: Zuflussleitung
- 128: Ablussleitung
- 129: Oberfläche
- 130: Klebefolie
- 140: Deckschicht

## Patentansprüche

1. Verfahren zum Herstellen einer Vielzahl von elektronischen Sensorvorrichtungen (10) zum Detektieren von chemischen oder biologischen Spezies (12, 12', 12"), mit den folgenden Schritten:
- Einbetten einer Vielzahl von jeweils aus einem Halbleitersubstrat vorgefertigten Halbleiter-Chips (30) in jeweils einem ersten Bereich einer Substratoberfläche (22) eines aus einem schmelzformbaren Material (26) hergestellten Substrats (20) durch:
- Positionieren der Vielzahl von vorgefertigten Halbleiter-Chips (30) auf einer Klebefolie so, dass jeweils eine Chipoberfläche (32) der vorgefertigten Halbleiter-Chips (30) der Klebefolie zugewandt ist, wobei jeder der Halbleiter-Chips (30) für eine oder mehrere Funktionen ausgebildet ist, die ausgewählt sind aus einer Gruppe, die folgendes umfasst: Verstärken bzw. Auswerten einer elektrischen Spannung, Verstärken bzw. Auswerten eines elektrischen Stroms, Verstärken bzw. Auswerten einer elektrischen Ladung und Verstärken bzw. Auslesen von Kapazitätsänderungen;
- Umspritzen der Vielzahl von auf der Klebefolie positionierten Halbleiter-Chips (30) mit dem schmelzformbaren Material (26) zum Bilden eines Moldmassen-Substrats; und
- Abnehmen der Klebefolie von dem Moldmassen-Substrat mit der Vielzahl darin eingebetteter Halbleiter-Chips (30);
- Aufbringen eines Fotolacks als Fotolack-Passivierungsschicht (28) auf das Moldmassen-Substrat mit der Vielzahl darin eingebetteter Halbleiter-Chips (30) und Öffnen der Fotolack-Passivierungsschicht (28) über vorbestimmten Kontaktflächen (38, 38') auf den Chipoberflächen (32) der Halbleiter-Chips (30);
- Aufbringen einer Vielzahl von Sensoreinrichtungen (40, 40') pro Halbleiter-Chip (30) und einer Vielzahl von elektrisch leitfähigen Verbindungen (44, 44') zwischen der Vielzahl von Sensoreinrichtungen (40, 40') und dem zugeordneten Halbleiter-Chip (30) auf der Fotolack-Passivierungsschicht (28) mittels einer Dünnschichttechnologie oder mittels eines Druckverfahrens;
- Vereinzeln der elektronischen Sensorvorrichtungen (10) aus dem Moldmassen-Substrat; und
- Ausbilden der Vielzahl von Sensoreinrichtungen (40, 40') der vereinzelten elektronischen Sensorvorrichtungen (10) mit jeweils einer aktiven Sensoroberfläche (42) auf bzw. in mindestens einem zweiten Bereich der Substratoberfläche (22) des aus dem schmelzformbaren Material (26) hergestellten Substrats (20), wobei die jeweilige aktive Sensoroberfläche (42) dazu ausgebildet wird, chemische oder biologische Spezies (12, 12', 12") zu detektieren und infolge einer für die Spezies charakteristischen Wechselwirkung mit der aktiven Sensoroberfläche (42) ein elektrisches Signal zu erzeugen, das aus einer Gruppe ausgewählt ist, die folgendes umfasst: eine elektrische Spannung, einen elektrischer Strom, eine elektrische Ladung und eine Kapazitätsänderung.

2. Verfahren zum Herstellen einer mikrofluidischen Vorrichtung (100) zum Detektieren von chemischen oder biologischen Spezies (12, 12', 12"), mit den folgenden Schritten:
- Herstellen einer elektronischen Sensorvorrichtung (10) gemäß dem Verfahren nach Anspruch 1,
- Bereitstellen eines ersten Polymersubstrats (110) mit einer strukturierbaren Oberfläche (111) und Ausbilden einer Ausnehmung (112) in dieser dadurch strukturierten Oberfläche (111),
- Anordnen der elektronischen Sensorvorrichtung (10) in der Ausnehmung (112),
- Bereitstellen eines zweiten Polymersubstrats (120) mit einer strukturierbaren Oberfläche (121) und Ausbilden einer Flusszelle (122) in dieser dadurch strukturierten Oberfläche (121),
- Auflegen des zweiten Polymersubstrats (120) auf das erste Polymersubstrat (110), wobei die strukturierte Oberfläche (121) des zweiten Polymersubstrats (120) der strukturierten Oberfläche (111) des ersten Polymersubstrats (110) zugewandt ist und die Flusszelle (122) des zweiten Polymersubstrats (120) angrenzend an die Ausnehmung (112) und die darin angeordnete elektronische Sensorvorrichtung (10) angeordnet ist, und
- flächig Verbinden der strukturierten Oberfläche (121) des zweiten Polymersubstrats (120) mit der strukturierten Oberfläche (111) des ersten Polymersubstrats (110) zumindest in Bereichen außerhalb der Ausnehmung (112) des ersten Polymersubstrats (110).

## Claims

1. Method for producing a multiplicity of electronic sensor apparatuses (10) for detecting chemical or biological species (12, 12', 12"), comprising the following steps:
- embedding a multiplicity of semiconductor chips (30) prefabricated in each case from a semiconductor substrate in a respective first region of a substrate surface (22) of a substrate (20) produced from a melt-mouldable material (26) by:
- positioning the multiplicity of prefabricated semiconductor chips (30) on an adhesive film such that a respective chip surface (32) of the prefabricated semiconductor chips (30) faces the adhesive film, wherein each of the semiconductor chips (30) is configured for one or more functions selected from a group comprising the following: amplifying and/or evaluating an electrical voltage, amplifying and/or evaluating an electrical current, amplifying and/or evaluating an electrical charge and amplifying and/or reading out capacitance changes;
- encapsulating the multiplicity of semiconductor chips (30) positioned on the adhesive film by injection moulding with the melt-mouldable material (26) in order to form a moulding compound substrate; and
- removing the adhesive film from the moulding compound substrate with the multiplicity of semiconductor chips (30) embedded therein;
- applying a photoresist as a photoresist passivation layer (28) on the moulding compound substrate with the multiplicity of semiconductor chips (30) embedded therein and opening the photoresist passivation layer (28) above predetermined contact areas (38, 38') on the chip surfaces (32) of the semiconductor chips (30);
- applying a multiplicity of sensor devices (40, 40') per semiconductor chip (30) and a multiplicity of electrically conductive connections (44, 44') between the multiplicity of sensor devices (40, 40') and the assigned semiconductor chip (30) on the photoresist passivation layer (28) by means of a thin-film technology or by means of a printing method;
- simulating the electronic sensor apparatuses (10) from the moulding compound substrate; and
- forming the multiplicity of sensor devices (40, 40') of the singulated electronic sensor apparatuses (10) with a respective active sensor surface (42) on and/or in at least one second region of the substrate surface (22) of the substrate (20) produced from the melt-mouldable material (26), wherein the respective active sensor surface (42) is configured to detect chemical or biological species (12, 12', 12") and, on account of an interaction with the active sensor surface (42) that is characteristic of the species, to generate an electrical signal selected from a group comprising the following: an electrical voltage, an electrical current, an electrical charge and a capacitance change.

2. Method for producing a microfluidic apparatus (100) for detecting chemical or biological species (12, 12', 12"), comprising the following steps:
- producing an electronic sensor apparatus (10) in accordance with the method according to Claim 1,
- providing a first polymer substrate (110) having a structurable surface (111) and forming a cutout (112) in this thereby structured surface (111),
- arranging the electronic sensor apparatus (10) in the cutout (112),
- providing a second polymer substrate (120) having a structurable surface (121) and forming a flow cell (122) in this thereby structured surface (121),
- placing the second polymer substrate (120) onto the first polymer substrate (110), wherein the structured surface (121) of the second polymer substrate (120) faces the structured surface (111) of the first polymer substrate (110) and the flow cell (122) of the second polymer substrate (120) is arranged in a manner adjacent to the cutout (112) and the electronic sensor apparatus (10) arranged therein, and
- areally connecting the structured surface (121) of the second polymer substrate (120) to the structured surface (111) of the first polymer substrate (110) at least in regions outside the cutout (112) of the first polymer substrate (110).

## Revendications

1. Procédé de fabrication d'une pluralité d'arrangements de détection (10) électroniques destinés à détecter des espèces chimiques ou biologiques (12, 12', 12"), comprenant les étapes suivantes :
- enrobage d'une pluralité de puces en semiconducteur (30) respectivement préfabriquées à partir d'un substrat semiconducteur respectivement dans une première zone d'une surface de substrat (22) d'un substrat (20) fabriqué à partir d'un matériau façonnable à l'état fondu (26) par :
- positionnement de la pluralité de puces en semiconducteur (30) préfabriquées sur un film adhésif de telle sorte qu'une surface de puce (32) des puces en semiconducteur (30) préfabriquées fait respectivement face au film adhésif, chacune des puces en semiconducteur (30) étant configurée pour une ou plusieurs fonctions qui sont choisies dans le groupe comprenant les suivantes : amplification ou interprétation d'une tension électrique, amplification ou interprétation d'un courant électrique, amplification ou interprétation d'une charge électrique et amplification ou relevé de variations de capacité ;
- surmoulage de la pluralité de puces en semiconducteur (30) positionnées sur le film adhésif avec le matériau façonnable à l'état fondu (26) en vue de former un substrat en masse moulée ; et
- enlèvement du film adhésif du substrat en masse moulée dans lequel sont enrobées la pluralité de puces en semiconducteur (30) ;
- application d'un vernis photorésistant en tant que couche de passivation en vernis photorésistant (28) sur le substrat en masse moulée comportant la pluralité de puces en semiconducteur (30) enrobées dans celui-ci et ouverture de la couche de passivation en vernis photorésistant (28) au-dessus de surfaces de contact (38, 38') prédéfinies sur les surfaces de puce (32) des puces en semiconducteur (30) ;
- application d'une pluralité de dispositifs de détection (40, 40') par puce en semiconducteur (30) et d'une pluralité de liaisons électriquement conductrices (44, 44') entre la pluralité de dispositifs de détection (40, 40') et la puce en semiconducteur (30) associéesur la couche de passivation en vernis photorésistant (28) au moyen d'une technologie à couche mince ou au moyen d'un procédé d'impression ;
- séparation des arrangements de détection (10) électroniques du substrat en masse moulée ; et
- formation de la pluralité de dispositifs de détection (40, 40') des arrangements de détection (10) électroniques séparés respectivement avec au moins une surface de détection active (42) sur ou dans au moins une deuxième zone de la surface de substrat (22) du substrat (20) fabriqué à partir du matériau façonnable à l'état fondu (26), la surface de détection active (42) respective étant configurée pour détecter des espèces chimiques ou biologiques (12, 12', 12") et, suite à une interaction caractéristique de l'espèce avec la surface de détection active (42), générer un signal électrique qui est sélectionné dans un groupe comprenant les éléments suivants : une tension électrique, un courant électrique, une charge électrique et une variation de capacité.

2. Procédé de fabrication d'un arrangement microfluidique (100) destiné à détecter des espèces chimiques ou biologiques (12, 12', 12"), comprenant les étapes suivantes :
- fabrication d'un arrangement de détection (10) électronique conformément au procédé selon la revendication 1,
- fourniture d'un premier substrat en polymère (110) ayant une surface (111) structurable et formation d'une cavité (112) dans cette surface (111) ainsi structurée,
- placement de l'arrangement de détection (10) électronique dans la cavité (112),
- fourniture d'un deuxième substrat en polymère (120) ayant une surface (121) structurable et formation d'une cellule d'écoulement (122) dans cette surface (121) ainsi structurée,
- dépose du deuxième substrat en polymère (120) sur le premier substrat en polymère (110), la surface (121) structurée du deuxième substrat en polymère (120) faisant face à la surface (111) structurée du premier substrat en polymère (110) et la cellule d'écoulement (122) du deuxième substrat en polymère (120) étant disposée de manière adjacente à la cavité (112) et à l'arrangement de détection (10) électronique placé dans celle-ci, et
- assemblage à plat de la surface (121) structurée du deuxième substrat en polymère (120) avec la surface (111) structurée du premier substrat en polymère (110) au moins dans les zones en-dehors de la cavité (112) du premier substrat en polymère (110).
